# EUROPÄISCHE PATENTSCHRIFT

(11) **EP 1 692 216 B1**
(45) Veröffentlichungstag und Bekanntmachung des Hinweises auf die Patenterteilung: **14.11.2012**
(21) Anmeldenummer: 04820065.3
(22) Anmeldetag: 13.12.2004
(51) Int. Cl.: C08J 5/18, C08J 3/24, A61K 9/00, A61K 9/70, C08B 15/00

(54) **DARREICHUNGSFORM BASIEREND AUF VERNETZTEN HYDROPHILEN POLYMEREN**
FORM OF ADMINISTRATION BASED ON CROSSLINKED HYDROPHILIC POLYMERS
FORME GALENIQUE A BASE DE POLYMERES HYDROPHILES RETICULES

(30) Priorität: 12.12.2003 DE 10358748
(43) Veröffentlichungstag der Anmeldung: 23.08.2006
(73) Patentinhaber: LTS LOHMANN Therapie-Systeme AG, 56626 Andernach (DE)
(72) Erfinder: BARTHOLOMÄUS, Johannes, 52080 Aachen (DE); VAZQUEZ LANTES, Maria Christina, 85635 Siegertsbrunn (DE)
(74) Vertreter: Siemund, Volker
(86) Internationale Anmeldenummer: PCT/EP2004/014147
(87) Internationale Veröffentlichungsnummer: WO 2005/056648

(56) Entgegenhaltungen:
- EP-A- 0 410 696
- WO-A-01/58430
- WO-A-98/22097
- WO-A-99/55312
- WO-A-03/063839
- US-B1- 6 375 963

## Beschreibung

Die vorliegende Erfindung betrifft ein Verfahren zur Herstellung einer filmförmigen Darreichungsform zur oberflächlichen Verabreichung wenigstens eines Wirkstoffes und/oder Nährstoffes an ein Lebewesen umfassend wenigstens eine wirkstoffhaltige und/oder nährstoffhaltige Schicht basierend auf mit mindestens einem Polyacrylsäurederivat vernetzten hydrophilen Polymeren durch Aufbau einzelner Schichten übereinander auf einer glatten Oberfläche,
wobei als Polyacrylsäurederivat eine Polyacrylsäure vernetzt mit Allylsucrose oder Allylpentaerythritol und/oder eine Polyacrylsäure vernetzt mit Divinylglycol, gegebenenfalls mit Calcium neutralisiert, verwendet wird und wobei als hydrophiles Polymer Hydroxypropylmethylcellulose, Hydroxyethylcellulose und/oder Methylcellulose eingesetzt wird.
gekennzeichnet durch die Schritte:
a) gleichzeitiges Versprühen einer wäßrigen Lösung der hydrophilen Polymere und des Wirkstoffes und/oder des Nährstoffes und einer wäßrigen Lösung des Polyacrylsäurederivats, wobei die hydrophilen Polymere und das Polyacrylsäurederivat in situ vernetzen,
b) Entfernen des Wassers durch Trocknen.

Filmförmige Darreichungsformen zur oberflächlichen Verabreichung weisen üblicherweise eine mehrschichtige Struktur auf und bestehen typischerweise aus einer Deckschicht, einer wirkstoffhalfigen und/oder nährstoffhaltigen Schicht und einer Haftschicht.

WO 98/22097 offenbart Filme, die in Wasser aufquellen und unlösliche Gele bilden. Die Herstellung der Filme aus Beispielen erfolgt ausschließlich durch Gießen mit anschließendem Trocknen. Eine Vernetzung wird nicht erwähnt. Aus der Offeniegungsschrift DE 199 32 603 ist bereits die Vernetzung von hydrophilen Polymeren mit Tannin zur Herstellung filmförmiger Darreichungsformen zur Verabreichung von Wirkstoffen und/oder Nährstoffen bekannt. Der Einsatz von Tannin kann der Darreichungsform sowohl eine gelbliche Farbe als auch einen nicht bekömmlichen Geschmack verleihen,

Daher stellte sich die Aufgabe, ein Verfahren zur Herstellung für eine filmförmige Darreichungsform basierend auf vernetzten hydrophilen Polymeren zur Verfügung zu stellen, bei der der zum Einsatz kommende Vernetzer die Darreichungsform nicht nachteilig verändert.

Diese Aufgabe wurde durch die Bereitstellung der erfindungsgemäßen, filmförmigen Darreichungsform zur oberflächlichen Verabreichung wenigstens eines Wirkstoffes und/oder Nährstoffes an ein Lebewesen umfassend wenigstens eine wirkstoffhaltige
und/oder nährstoffhaltige Schicht basierend auf vernetzten hydrophilen Polymeren gelöst, die dadurch gekennzeichnet ist, daß die hydrophilen Polymere mit mindestens einem Polyacrylsäurederivat nach dem beanspruchten Verfahren vernetzt worden sind.

Als Polyacrylsäurederivat für die Vernetzung von hydrophilen Polymeren eignen sich ohne Einschränkung alle pharmazeutisch akzeptable Polyacrylsäurederivate, wie beispielsweise eine gegebenenfalls vernetzte Polyacrylsäure, bevorzugt eine Polyacrylsäure vernetzt mit Allylsucrose oder Allylpentaerythritol (Carbomer nach USP-NF) und/oder eine Polyacrylsäure vernetzt mit Divinylglycol , gegebenenfalls mit Calcium neutralisiert (Polycarbophil nach USP-NF). Besonders bevorzugt ist eine mit Divinylglycol vernetzte Polyacrylsäure.

Als hydrophile Polymere für die erfindungsgemäße, filmförmige Darreichungsform eignen sich insbesondere wasserlösliche Celluloseether, bevorzugt Hydroxypropylmethylcellulose, Hydroxyethylcellulose und/oder Methylcellulose, besonders bevorzugt Hydroxypropylmethylcellulose.

Durch den Einsatz von Polyacrylsäurederivaten werden Darreichungsformen erhalten, deren mechanischen Eigenschaften denen von Darreichungsformen mit Tannin als Vernetzer vergleichbar sind.

So ist durch die Vernetzung der filmbildenden hydrophilen Polymeren mit Polyacrylsäurederivate eine ausreichend sichere Handhabung der filmförmigen Darreichungsform, z. B. beim Herausnehmen aus der Verpackung und Einbringen auf den Applikationsort, ohne Beschädigung der Darreichungsform durch Zerreißen gewährleistet und eine schnelle Auflösung der Darreichungsform am Appilkationsort, z. B. an einer nassen Schleimhaut, verhindert.

Die nach dem erfindungsgemäßen Verfahren hergestellten filmförmige Darreichungsform wird zur oberflächlichen Verabreichung wenigsterts eines Wirkstoffes und/oder Nährstoffes an ein Lebewesen eingesetzt.

Bezüglich der in der wirftstoffhaltigen und/oder nährstoffhaltigen Schicht enthaltenen Wirkstoffe und/oder Nährstoffe gibt es prinzipiell keine Einschränkung. Vorzugsweise sind die Wirkstoffe bzw. Nährstoffe jedoch Duftstoffe, Aromen, Diagnostica, Pflanzenschutzmittel, pharmazeutische Wirkstoffe, Vitamine. Düngemittel und/oder andere Nährstoffe.

Als pharmazeutische Wirkstoffe können Analgetica, Antiallergica, Antibiotica, Antiemetica, Antiseptica, Antihisteminica, Antihypertonia, Appetitzügler, Cardiaca, Chemotherapeutica, Enzympräparate, Hormone, Immunmodulatoren, Impfungen, Lokelanästhetica, Psychopharmaka, Spasmolytica, Virustatica, Vitamine und Zytostatica verwendet werden.

Geeignete Wirkstoffe sind insbesondere Diamorphin, Alflentanil, Sufentanyl, Pentazocin, Buprenorphin, Nefoparn, Flupirtin, Tramadol, Oxycodon, Metamizol, Propyphenanzon Phenazone, Nifenazon, Phenylbutazon, Oxyphenbutazon, Mofebutazon, Diffunisal, Meptazinol, Methadon, Pethidin, Meloxicam, Fenbufen, Mefenamisäure, Tenoxicam, Azapropazon, Piritramid, Tramadol, Amantadin, Benzottopin, Procyclidin, Moclobemid, Tranylcypromid, Maprotilin, Doxepin, Opipramol, Desipramin, Imipramin, Fluroxamin, Paroxetin, Trazodon, Viloxazin, Fluphenazin, Perphenazin, Promethazin, Thioridazin, Triflupromazin, Prothipendyl, Tiotixen, Chlorproathixen, Pipamperon, Pimozid, Fenethyllin, Trifluoperazin, Thioridazin, Oxazepam, Alprazolam, Clobazam, Piracetam, Melfalan, Cyclophosphamid, Trofosfamid, Chlorambucil, Lomustin, Busilfan, Prednimustin, Mercaptopurin, Thioguanin, Hydroxycarbamid, Altretamin, Procarbazin, Lisurid, Methysergid, Pizotifen, Roxatidin, Pirenzipin, Proglumid, Bromoprid, Pheniramin, Dimethinden, Tritoqualin, Loratadin, Doxylamin, Mequitazin, Dexchlorpheniramin, Triprolidin, Oxatomid, Moxonidin, Doxazosin, Urapidil, Dihydralazin, Deserpidin, Alprenolol, Bupranolol, Penbutolol, Esmolol, Ciliprolol, Motipranolol, Nadolol, Quinapril, Fosinopril, Cilazapril, Democlocyclin, Lymecyclin, Oxytetracyclin, Sulfamethopyrazin, Aerosoxacin, Becampicillin, Piperacillin, Pivampicillin, Cloxacillin, Flucloxacillin, Metronidazol, Clindamycin, Cefaclor, Cefpodoxim, Cephalexin, Cefradin, Pirbuterol, Orciprenalin, Clenbuterol, Procaterol, Cholintheophyllinat, Theophyllin-ethylenediamin, Ketofen, Viquidil, Procainamid, Mexiletin, Tocainid, Ipratropium, Tobutamid, Gliquidon, Gliborurid, Tolazamid, Acarbose und pharmazeurisch aktive Salze oder Ester der vorgenannten Wirkstoffe sowie Kombinationen von zwei oder mehreren dieser Wirkstoffe oder deren Salze oder Ester.

Geeignete Wirkstoffe sind beilspielsweise Acebutolol, Acetylcystein, Acetylsalicylsäure, Aciclovir, Albrazolam, Alfacalcidol, Allantoin, Allopurinol, Ambroxiol, Amikacin, Amiloride, Aminoessigsäure, Amiodaron, Amitriptylin, Amlodipin, Amoxicillin, Ampicillin, Ascorbic Acid, Aspartam, Astemizole, Atenolol, Beclometason, Benserazid, Benzalkonium Hydrodrochlorid, Benzocain, Benzoesäure, Betametasone, Bezafibrate, Blotin, Biperiden, Bisoprolol, Bromacepam, Bromhexine, Bromocriptine, Budesonide, Bufexamac, Buflomedil, Buspirone, Coffein, Campher, Captopril, Carbamacipine, Carbidopa, Carboplatin, Cefaclor, Cefalexin, Cefadroxil, Cefazolin, Cefixime, Cefotaxim, Ceftazidin, Ceftriaxon, Cefuroxim, Celedilin, Chloramhenicol, Chlorhexidin, Chlorpheniramin, Chlortalidon, Cholin, Ciclosporin, Cilastatin, Cimetidin, Ciprofloxacin, Cisaprid, Cisplatin, Clarithromycin, Clavulansäure, Clomibramin, Clonazepam, Clonidin Clotrimazol, Codein, Colestyramin, Cromoglicinsäute, Cyanocobalamin, Cyproteron, Desogetrel, Dexamethason, Dexpanthenol, Dexthromethorphan, Dextropropoxiphen, Diazepam, Diclofenac, Digoxin, Dihydrocodein, Dihyderoergotamin, Dihydroergotoxin, Diltiazem, Diphenhydramin, Dipyridamol, Dipyron, Disopyramid, Domperidon, Dopamin, Doxcyclin, Enalapril, Ephedrin, Epinephrine, Ergocalciferol, Ergotamin, Erytrhomycin, Estradiol, Ethinylestradinol, Etoposid, Famotidin, Felodipin, Fenofbrat, Fenoterol, Fentanyl, Flavin Mononucleotid, Fluconazol, Flunarizin, Fluorouracil, Fluoxetin, Flurbiprofen, Furosemid, Gailopamil, Gemfibrozil, Gentaminicin, Ginkgo Biloba, Glibenclamid, Glipizid, Glozapin, Glycyrrhiza Glabra, Groseofulvin, Guaifenesin, Haloperidol, Heparin, Hyaluronsäure, Hydrodilorothiazid, Hydrocodon, Hydrocortison, Hydromorphon, Ibratropium Hydroxide, Ibuprofen, Imipenem, Indomethacin, lohexol, Iopamidol, Isosorbid Dinitrat, Isosorbid Mononitrat, Isotretionin, Ketotifen, Ketoconazol, Ketoprofen, Ketorolac, Labatalon, Lactulose, Lecithin, Levocamitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Monoxidil, Misoprostol, Morphin, Multivitamine und Mineralien, N-Methylephedim, Naftidrofuril, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Pherlobarbitel, Phenoxifyllin, Phenoxymethylpenicillin, Phenylephrin, Phenylpropanolamin, Phenytoin Piroxicam, Polymxyxin B, Povidone-Iod, Pravastatin, Prazepam, Prazosin, Prednisolon, Prednison, Propafenon, Propranolol, Proxyphyllin, Pseudoephedrin, Pyridoxine, Chinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracycin, Theophyllin, Thiamin, Ticlopidin, Timolol, Tranexamsäure, Tretinoin, Triamcinolone Acetonide, Triamteren, Trimethoprim, Troxerutin, uracil, Valproinsäure, Vancomycin, Verapamil, Vitamine E, Zidovudine.

Weiterhin geeignete Wirkstoffe sind Proclorperazin-edisylal, Elsen-II-sulfat, Aminocapronsäure, Kaliumchlorid, Mecamylamin-hydrihlorid, Procainamidhydrochlorid, Amphetamin-sulfat, Benzphetamin-hydrochlorid, Isoporterenol-sulfat, Methamphetamin-hydrochlorid, Phenmetrazin-hydrochlorid, Bethanecholchlorid, Methacholin-chlorid, Pilocarpin-hydrochlorid, Atropinsulfat, Methascopolamin-bromid, Isopropamid-iodid, Tridihexethylchlorid, Phenformin-hydrochlorid, Methylphenidathydrochlorid, Oxprenolol-hydrochlorid, Metroprolol-tartrat, Cimetidin-hydrochlorid, Diphenidol, Meclizin-hydrochlorid, Proclorperazinmaleat, Phenoxybenzamin, Thiethylperazin-maleat, Anisindon, Diphenadion, Erythrit-tetranitrat, Dizoxin, Isofurophat, Acetazolamid, Mathazolamid, Bendroitumethiazid, Chlorpropamid, Tolazamid, Chlormadinon-acetat, Phenaglycodol, Aluminium-aspirin, Methotrexat, Acetyl-sulfioxazol, Progestine, österrogene Steroide, progestatine Steroide, Corticosteroide, 17-β-östradiol, Ethinyl-östradiol-3-methyl-ester, Hydrocorticosteronacetat, Methyltesteron, 17-α-Hydroxyprogesteron-acetat, 19-Nor-progesteron, Norethindron, Progesteron, Norgesteron, Norethynodrei u. a.

Weitere Beispiele von Wirkstoffen sind Fenoprofen, Sulindac, Indoprofen, Nitroglycerin, Timolol, Alprenolol, Imipramin, Chlorpromazin, Dihydroxyphenylalanin, Pivaloxyloxyethylester von α-Methyldopa-hydrochlorid, Calcium-gluconat, Eisen-IIlactat, Vincamin, Phenoxybenzamin, Blocker u. ä. Die Wirkstoffe sind bekannt aus "Pharmaceutical Sciences" von Remington, 14. Auflage, 1979, Mack Publishlng Co., Easton, Pennsylvania; "The Drug, The Nurse, The Patient, Including Current Drug Handbook", 1974-1976, von Falconer et al, Saunder Co., Philadelphia, Pennsylvania, und "Medical Chemistry", 3. Auflage, Band 1 und 2, von Burger, Wiley-Interscience, New York.

Repräsentative Arzneimittel, die an warmblütige Tiere, beispielsweise Wiederkäuer, mit Hife der erfindungsgemäßen Darreichungsform verabreicht werden können, sind u. a. Antheimintica, wie Mebendazol, Levamisol, Albendazol, Cambendazol, Fenbendazol, Parbendazol, Oxfendazol, Oxybendazol, Thiabendazol, Tichlorfon, Praziquantel, Morantel und Pirantel, u. ä.; Antiparasiten-Mittel, wie Avermectine und Ivermectin, wie in den US-PS 41 99 569 und 43 89 397 (Merck) und in "Science", Band 221, S. 823 - 828, 1983 angegeben, wo diese Ivermectin-Antiparasiten-Mittel als geeignet zur Unterstützung bei der Bekämpfung von üblicherweise bei Säugetieren auftretenden Würmern, wie Rundwürmem (Spulwürmern), Lungenwürmern u. ä., angegeben sind, und auch daß das Ivermectin geeignet ist zur Behandlung von Insekteninfektionen, wie Maden, Läusen, Milbenräude u. ä.; antimikrobielle Mittel, wie Chlortetracyclin, Oxytetracyclin, Tetracyclin, Gentamicin, Streptomycin, Dihydrostreptomycin, Bacitracine, Erthromycin, Ampicilline, Penicilline, Cephalosporine u. ä.; Schwefel enthaltende Arznelmittel (sufa drugs), wie Sulfamethazin, Sulfathiazol u. ä.: Wachstumsstimulantien, wie Monesin®-natrium und Elfazepam®; Anti-Flohmittel (defleaing agents), wie Dexamethazon und Flumethazon; die Verdauung im Pansen beeinflussende Mittel und Ionophore, wie Lasalocid, Virginamiycin, Salinomycin und Ronnel; Mineralstoffe, wie Kupferoxid, Cobaltsulfat, Kallumiodat, Zinkoxid, Mangansulfat, Zinksulfat, Selen, Natriumselenit, günstige Mineralsalze u. ä.; Antiblähmittel, wie organische Polysiloxane; hormonelle Wachstumszusätze, wie Stilböstrol; Vitamine, wie Vitamine A und D; mit 500 000 : 100 000 IU/f, Vitamin E mit 500 000 IU/f u, ä.; Antienteritlsmittel, wie Furazolidon, Wachstumsfaktoren, Nährstoffzusätze, wie Lysinmonohydrochlorid, Methionin, Magnesiumcarbonat u. ä.; β-Agonistan, Elenbuterol u. ä., und chemische Markierungsstoffe, wie Chromoxid, und Salze von Ytterbium und Erblum.

Die lokal wirkenden Wirkstoffe umfassen weiterhin Fungizide wie Amphotericin B, Antibiotika, wie Penicilline, Cephalosporine, Erythromycin, Tetracyclin, Aminoglycoside, antivirale Verbindungen wie Acyclovir, Idoxuridin, Atemverbesser wie Chlorophyil, Gewebewachstums hemmende Verbindungen, Antikariesverbindungen wie Metallfluoride, insbesondere Natriummoriofluorphosphat, Zinnfluorid, Aminfluorid, Schmerzmittel wie Methylsalicylat, Lokalanästhetika wie Benzocain, orale Antiseptika wie Chlorhexidin und dessen Salze, Hexylresorcin, Dequalinium Chlorid, Cetylpyridin Chlorid), Antientzündungsmittel, Hormone wie Oestriol, Antiplaqueverbindungen wie Chlorhexidin und dessen Salze, Octenidin, oder Mischungen von Thymol, Menthol, Methysalicylat, Eucalyptol, Pufferverbindungen wie Kallumphosphat, Calciumcarbonat, Natriumbicarbonat, Natrium- und Kaliumhydroxid sowie Desenelbilisatoren für Zähne wie z. B. Kaliumnitrat.

Desweiteren sind als aktive Wirkstoffe Desinfektiva wie Chlorverbindungen, insbesondere Calciumhypochlorit, ein Insektizid, Pestizid, Herbizid, Fungizid, oder Wachstumförderer bzw. Düngemittel wie z. B. Stickstoff haltige Verbindungen, insbesondere Harnstoff, Hamstofformaldehydverbindungen, Caliumnitrat, Caliumsulfat, Caliumchlorid, Ammoniumnitrat, Ammoniumsuflat, Monoammoniumphosphat, dibasisches Ammoniumphosphat, Ammoniumphosphorsäureverbindungen, Spurenelemente für Nahrungsmittel wie Eisen, Zink, Mangan, Kupfer, Bor, Molybden oder Mischungen daraus geeignet.

Wirkstoffe, die sich für die erfindungsgemäße Darreichungsform eignen, sind auch Steroidhormone wie:

Gestagen wirksame Steroidharmone, wie beispielsweise das 13-Ethyl-17β-hydroxy-18,19-dinor-17α-pregn-4-en-20yl-3-on, das 13-Ethyl-17β-hydroxy-18,19-dinor-17α-pregna-4,15-dien-20yn-3-on (=Gestoden), das 13-Ethyl-17β-hydroxy-11-methylen-18,19-dinor-17α-pregn-4-en-20yn oder das 13-Ethyl-11-methylen-17β-hydroxy-18,19-dinor17α-pregn-4-en-3-on (3-Keto-Desogestrel) Estrogen wirksame Steroidhormone das 3-Hydroxy-1,3,5-(10)-estratrien-17-on (=Estron), das 1,3,5(10)-Estratrien-3,17β-diol oder das 1,9-Nor-17α-pregna-1,3,5(10)-trien-20yn-3,17β-diol, das 17β-Hydroxy-19-nor-17α-pregn-4en-20yn-3-on, das 14α, 17α-Ethano-1,3,5(10)-estratrien-3,17β-diol (=Cyclodiol) und das 14α, 17α-Ethano-1,3,5(10)-estratrien-3,16α,17β-triol (=Cyclotriol) und Kombinationen dieser Gestagen und Estrogene.

Androgen wirksame Steroidhormone wie das 17β-Hydroxy-4-androsten-3-on (=Testosteron) und dessen Ester oder das 17β-Hydroxy-1α-methyl-5α-androsten-3-on (=Mesterolon).

Antiandrogen wirksame Steroidhormone wie das 17α-Acetoxy-6-chlor-1β,2β-dihydro-3H-cyclopropa[1,2]-pregna-1,4,6-trien-3,20-dion.

Kortikoide wie das 11β,17α,21-Trihydroxy-4-pregnen-3,20-dion, das 11β,17α,21-Trihydroxy-1,4-pegnadien-3,20-dion, das 11β,17α,21-Trihydroxy-6α-methyl-1,4-pregnatrien-3,20-dion und das 6α-Fluor-11β,21-dihydroxy-16α-methyl-1,4-pregnadien-3,20-dion (=Diflucortolon) und deren Ester.

Geeignete Wirkstoffe sind fernen
Ergolin-Derivate, wie das Lisurid, [3-(9,10-Didehydro-6-methyl-8α-ergolinyl)-1,1-diethylharnstoff], das Bromlisurid [=3-(2-Brom-9,10-dehydro-6-methyl-8α-ergolinyl-1,1-diethylharnstoff], das Tergurid [=3-(6-Methyl-8α-ergolinyl-1,1-diethylharnstoff] und das Protergurid [=3-(6-Propyl-8α-ergolinyl)-1,1-diethylharnstoff].

Antihypertonika wie das 7α-Acetylthio-17α-hydroxy-3-oxo-4-pregnen-21-carbonsäure-γ-lacton und das 7α-Acetylthio-15β-,16β-methylen-3-oxo-17α-pregna-1,4-dien-21,17-carbolactan (=Mespirenon).

Antikoagulantia wie die 5-[Hexahydro-5-hydroxy-4-(3-hydroxy-4-methyl-1-octen-6-ynyl)-2(1H)-pentalenyliden)]-pentansäure (=Iloprost) oder die (Z)-7-[(1 R,2R,3R,5R)-5-Chlor-3-hydroxy-2-[(E)-(3R)-3-hydroxy-4,4-dimethyl-1-octenyl]-cyclopentyl]-5-heptensäure (=Nocloprost).

Psychopharmaka wie das 4-(3-Cyclopentyloxy-4-methoxy-phenyl-2-pyrrolidon (=Rolipram) und das 7-Chlor-1,3-dihydro-1-methyl-5-phenyl-2H-1,4-benzodiazepin-2-on.

Die Herstellung der wirkstoffhaltigen und/oder nährstoffhaltigen Schicht der erfindungsgemäßen Darreichungsform erfolgt vorzugsweise durch in-situ Vernetzung mit Polyacrylsäurederivaten während der Schichtbildung. Geeignet ist ein Gewichtsverhältnis von hydrophilen Polymeren zu Polyacrylsäurederivat(en) von 5:1 zu 5:4, besonders geeignet ist ein Gewichtsverhältnis von 5:2 bis 5:3.

Die nach dem erfindungsgemäßen Verfahren hergestellten filmförmigen Darreichungsformen können mehrschichtig sein. Sofern die filmförmigen Darreichungsformen mehrschichtig sind, können sie mehr als eine wirkstoffhaltige und/oder nährstoffhaltige Schicht, eine Deckschicht und gegebenenfalls eine Haftschicht aufweisen.

In der nach dem erfindungsgemäßen Verfahren hergestellten filmförmigen Darreichungsform basiert/basieren die wirkstoffhaltige(n) und/oder nährstoffhaltige(n) Schicht(en) auf mit Polyacrylsäurederivaten vernetzten hydrophilen Polymeren. Die wirkstoffhaltige(n) und/oder nährstoffhaltig(n) Schicht(en) kann/können den Wirkstoff in einer molekulären und/oder partikulären Form enthalten.

Aus der wirkstoffhaltigen und/oder nährstoffhaltigen Schicht bzw. den weiteren vorhandenen wirkstoffhaltigen und/oder nährstoffhaltigen Schichten kann die Wirkstoff- und/oder Nährstoff- Freisetzung nicht nur über die unterschiedliche Wirkstoff- und/oder Nährstoff- Konzentration, sondern auch über den Grad der Vernetzung der hydrophilen Polymeren gesteuert werden. Innerhalb einer wirkstoffhaltigen und/oder nährstoffhaltigen Schicht kann beispielsweise über einen Konzentration-Gradienten des Wirkstoffs und/oder des Nährstoffe die Freisetzung gesteuert werden. Eine weitere Möglichkeit, die Wirkstoff- und/oder die Nährstoff-Freisetzung zu beeinflussen, besteht darin, mehrere wirkstoffhaltige und/oder nährstoffhaltige Schichten mit unterschiedlichen Wirkstoff- und/oder Nährstoff-Konzentrationen in den erfindungsgemäßen filmförmigen Darreichungsformen vorzusehen, Weiterhin können auch wirkstofffreie bzw. nährstofffreie Schichten, ggf. aus vernetzten hydrophilen Polymeren, zwischen den wirkstoffhaltigen bzw. nährstoffhaltigen Schichten vorliegen. So kann damit aus einer ersten wirkstoffhaltigen auf hydrophilen Polymeren basierenden Schicht der Wirkstoff schnell und in einer ausreichenden Menge zur Erzielung einer unmittelbaren Wirkung freigesetzt werden, während aus weiteren wirkstoffhaltigen Schichten eine länger andauernde Wirkstoff-Freisetzung zur Erzielung einer anhaltenden Wirkung ernöglicht wird.

Die wirkstoffhaltige und/oder nährstoffhaltige Schicht weist vorzugsweise eine Dicke von 30-500 µm auf.

Die nach dem erfindungsgemäβen verfahren hergestellten filmförmige Darreichungsform weist vorzugsweise eine Deckschicht auf. Die Deckschicht besteht vorzugsweise aus einem wasserunlöslichen Polymer und ist für den Wirkstoff und/oder Nährstoff undurchlässig. Damit wird eine unidirektionale Wirkstoff- und/oder Nährstoff-Freisetzung gewährleistet. Bei dieser unidirektionalen Freisetzung wird der Wirkstoff und/oder Nährstoff nur an den Applikationsort freigesetzt.

Die Deckschicht besteht aus wenigstens einem wasserunlöslichen Celluloseether, vorzugsweise aus Alkylcellulose, besonders bevorzugt aus Ethylcellulose, oder einem wasserunlöslichen Celluloseester, vorzugsweise Celluloseacetat, und/oder einem wasserunlöslichen Poly(meth)acrylat, vorzugsweise einem Poly(C1-4)-alkyl(meth)acrylat, Poly(C1-4)-dialkylamino-(C1-4)alkyl(meth)acrylat und/oder deren Copolymere, ganz besonders bevorzugt einem Copolymer aus Ethylacrylat/Methylmethacrylat und/oder einem Copolymer aus Ethyacrylat/Methylmethacrylat/Trimethylammoniummethymethacrylat-Chlorid. Gegebenenfalls kann die Deckschicht neben Celluloseethem, Celluloseestern und/oder Poly(meth)acrylaten auch Weichmacher enthalten.

In einer bevorzugten Ausführungsform der beanspruchten Erfindung ist die Deckschicht aus Ethylcellulose oder aus einem Copolymer aus Ethylacrylat/Methylmethacrylat/Trimethylammoniumethylmethacryfat-Chlorid mit einem molaren Verhältnis der jeweiligen Monomeren von 1 : 2 : 0,1, in beiden Fällen mit einer prozentualen Menge am Weichmacher, bevorzugt Triethylcitrate, von 20 bis 40 Gew. % bezogen auf die Menge an Polymer. Ganz besonders bevorzugt ist eine Deckschicht bestehend aus einem Copolymer aus Ethylacrylat/Methylmethacrylat mit einem molaren Verhältnis der jeweiligen Monomeren von 2 : 1 (Weichmacher Zusatz in diesem Fall ist nicht unbedingt erforderlich).

Die Deckschicht weist vorzugsweise eine Dicke von 10 bis 100µm auf.

Um eine bessere Haftung der erfindungsgemäßen Darreichungsform bei transmukosaler bzw. transdermaler Applikation zu gewährleisten, kann eine zusätzliche Schicht als Haftschicht in der erfindungsgemäßen Darreichungsform vorgesehen werden, die ausschließlich aus Polyacrylsäurederivaten besteht, beispielsweise aus einer gegebenenfalls vernetzten Polyacrylsäure, bevorzugt einer Polyacrylsäure vernetzt mit Allylsucrose oder Allylpentaerythritol (Carbomer nach USP-NF) und/oder einer Polyacrylsäure vemetzt mit Divinylglycol, gegebenenfalls mit Calcium neutralisiert (Polycarbophil nach USP-NF). Besonders bevorzugt ist hier eine mit Divinylglycol vernetzte Polyacrylsäure.

Die Haftschicht weist vorzugsweise eine Dicke von 10 bis 100 µm auf.

Üblticherweise reicht aber der Einsatz des Vernetzers aus Polyacrylsäurederivaten aus, um eine ausreichende Haftung der wirkstoffhaltigen Schicht zu erzielen.

Die nach dem erfindungsgemäßen Verfahren hergestellten filmförmige Darreichungsform kann vor der Applikation mit einer Schutzschicht bedeckt sein.

Die filmförmige Darreichungsform wird hergesteilt, indem die wirkstoffhaltige und/oder nährstoffhaltige Schicht bzw. die Wirkstoffhaltigen und/oder nährstoffhaltigen Schichten, vorzugsweise aus einer wässrigen Lösung der hydrophilen Polymeren und des Wirkstoffes und/oder des Nährstoffes durch Auftragen unter gleichzeitiger oder nachträglicher Einwirkung des Polyacrylsäurederivats als Vernetzer, vorzugsweise als wässrige Lösung, und Entfernung des Wassers durch Trocknen gebildet wird.

Auf die getrocknete wirkstoffhaltige und/oder nährstoffhaltige Schicht kann durch Aufbringen einer wässrigen Dispersion wie einer Latex bzw. Pseudolatex-Dispersion eines wasserunlöslichen Polymeren oder einer Lösung eines solchen Polymeren in einem geelgneten, organischen Lösungsmittel unter anschließender Entfernung des Wassers oder organischer Lösungsmittel durch Trocknen und/ oder Vakuumbehandlung die Deckschicht hergestellt werden.

Sofern bei der nach dem erfindungsgemäßen Verfahren hergestellten filmförmigen Darreichungsform eine Haftschicht vorhanden ist, wird diese vorzugsweise aus einer wässrigen Lösung bzw. Dispersion von gegebenenfalls vernetzten Polyacrylsäuren.

Vorzugsweise wird die filmförmige Darreichungsform hergesteilt, indem die einzelnen Schichten übereinander auf einer glatten Oberfläche aufgebaut werden, wobei das jeweils filmbildend Polymer zusammen mit dem gegebenenfalls vorhandenen Vernetzer und dem gegebenenfalls vorhandenen Wirkstoff und/oder Nährstoff pro Schicht jeweils durch Versprühen und Trocknen als Teilschichten aufgebracht wird. Die Trocknung erfolgt dabei vorzugsweise simultan mit dem Versprühen. Die Teilschichten haben vorzugsweise eine Dicke von 0,1 bis 10 µm.

Das Versprühen der wäßrigen Lösung von hydrophilen Polymeren und der wäßrigen Lösung des Vernetzers erfolgt nach dem beanspruchten Verfahren gleichzeitig, wobei sich die hydrophilen Polymere und der Vernetzer nach dem Versprühen vermischen und danach in situ das Polymer vernetzt wird.

Sofern in einer Schicht der Wirkstoff und/oder Nährstoff vorhanden ist, erfolgt die Beladung vorzugsweise dadurch, dass der Wirkstoff und/oder Nährstoff in der wässrigen Lösung von hydrophilen Polymeren bereits gelöst ist, bevor diese Lösung mit der Lösung des Vernetzers zusammengebracht wird.

Die große Variabilität dieser Verfahrensweise erlaubt es, den Schichtaufbau in beliebiger Reihenfolge durchzuführen. So kann zuerst die Haftschicht, wenn vorhanden, oder zuerst die Deckschicht als Grundlage für die darauffolgenden Schichten gebildet werden.

Vorzugsweise wird zur Durchführung des Herstellungsverfahrens eine Apparatur wie in DE 101 46 251 beschrieben eingesetzt. Die entsprechende Offenbarung gilt als Teil der vorliegenden Offenbarung.

Diese Vorrichtung umfaßt mindestens eine Sprühvorrichtung, einen Trockner und mindestens eine Platte, die zyklisch unter der Sprühvorrichtung hindurch bewegt wird. Vorzugsweise weist die Vorrichtung mehrere Düsen auf, deren Sprühkegel sich überlappen.

### Methode zur Bestimmung der Relsfestigkeit

Zur Bestimmung der Reißfestigkeit wird einen Texture Analyser TA.XT2I der Firma Winopal (Deutschland) eingesetzt. Filmstücke der Wirkstoffhaltigen und/oder nährstoffhaltigen Schicht mit einer Länge von 9,5 cm und einer Breite von 1 cm werden mit Einspannbacken an beiden Enden eingeklemmt und leicht eingespannt, so dass die freie Spannlänge 7 cm beträgt. Die Einspannbacken sind mit Beschichtungen auf der Oberfläche, die mit den Stücken in Kontakt kommen, versehen, um ein vorzeitiges Zerreißen der Stücke an den Kammern zu vermeiden. Falls ein Stück trotz Beschichtungen an den Klammern zerreißen sollte, werden diese Werte nicht berücksichtig. Mit einer konstanten Geschwindigkeit von 0,5 mm/s zieht die obere Klammer aufwärts. Die dabei zu jeder Zeitpunkt eingesetzte Kraft sowie die resultierende Dehnung wird von dem Texture Analyser aufgenommen. Die Kraft, die Dehnung und die Zeit werden dann mit der Hilfe einer Software dargestellt und analysiert.

Die Reißfestigkeit eines untersuchten Filmstückes ist die Kraft, die in dem Moment, in dem das jeweilige Stück zerreißt, gerade auf das Filmstück einwirkt.

### Beispiele

### Beispiel 1

a) Zur Herstellung der wirkstoffhaltigen Schicht wurde eine Lösung von 10 g Hydroxypropylmethylcellulose, 1 g des Wirkstoffes Prednisolen und 489 g Wasser sowie eine Lösung aus 2 g Polycarbophil in saurer Form in 498 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt bis eine Schichtdicke der wirkstoffhaltigen Schicht von 200 µm erreicht worden war.
b) Zur Herstellung der Deckschicht wurde ein durch Verdünnung von 333,33 g eines 30 %-igen wässrigen Latex mit 666,67 g Wasser erhaltenes 10 %-iges wässriges Latex aus einem Copolymer Ethylacryl/Methylmethacrylat mit einem molaren Verhältnis der Monomeren von 2:1, eingesetzt. Diese Dispersion wurde mit Hilfe der in DE 101 46 251 beschriebenen Apparatur in einem mehrmaligen Sprühvorgang, bei dem Jeweils die Teilschichten erzeugt wurden, bis eine Schichtdicke der Deckschicht von 50 µm erreicht worden war.

Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 2

In der selben Art und Weise wie in Beispiel 1 beschrieben, wurde eine Darreichungsform hergestellt, mit dem Unterschied, dass vor der wirkstoffhaltigen Schicht eine Haftschicht aufgetragen wurde, indem eine Lösung aus 6 g Polyacrylsäure vernetzt mit Divinylglycol (Polycarbophil®) in 494 g Wasser aufgesprüht wurde, bis eine Schichtdicke von 50 µm erreicht worden war.

Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 3

In der selben Art und Weise wie in Beispiel 1 beschrieben, wurde eine Darreichungsform hergestellt, mit dem Unterschied, dass 4 g statt 2 g Polycarbophil und entsprechend 496 g Wasser eingesetzt wurden.

Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 4

In der selben Art und Weise wie in Beispiel 1 beschrieben, wurde eine Darreichungsform hergestellt, mit dem Unterschied, dass 6 g statt 2 g Polycarbophil und entsprechend 494 g Wasser eingesetzt wurden.

Die auf diese Weise hergesteilte Darreichungsform war leicht zu handhaben und leicht auf die menschliche Haut sowie auf die menschlichen Schleimhäute, zum Beispiel auf die buccale Schleimhaut, aufzubringen.

Alle wirkstoffhaltigen Schichten der Beispiele 1 bis 4 wiesen jeweils eine Reißfestigkeit von über 40 N auf, die nach der vorstehend angegebenen Methode bestimmt wurde.

### Beispiel 5

In der selben Art und Weise wie in Beispiel 1 beschrieben, wurde eine Darreichungsform hergestellt, mit dem Unterschied, dass hier 8 g statt 2 g Polycarbophil und entsprechend 492 g Wasser eingesetzt wurden.

Die auf diese Weise hergestellte Darreichungsform war leicht zu handhaben und leicht auf die menschliche Haut sowie auf die menschlichen Schleimhäute, zum Beispiel auf die buccale Schleimhaut, aufzubringen.

### Beispiel 6 (nicht erfindungsgemäß)

Zur Herstellung einer wirkstofffreien Schicht, deren Farbe und Geschmack zu untersuchen war, wurde eine Lösung von 10 g Hydroxypropylmethylcellulose und 490 g Wasser sowie eine Lösung aus 2,5 g Polycarbophil in saurer Form in 498 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt bis eine Schichtdicke der wirkstoffhaltigen Schicht von 200 µm erreicht worden war.

Die auf diese Weise hergestellte wirkstofffreie Schicht weist weder eine gelbliche Farbe noch einen unangenehmen Geschmack auf.

### Vergleichsbeispiel 1

Zur Herstellung einer wirkstofffreien Schicht, deren auf Farbe und Geschmack zu untersuchen war, wurde eine Lösung von 10 g Hydroxypropylmethylcellulose und 490 g Wasser sowie eine Lösung aus 2,5 g Tannin in 498 g Wasser hergestellt. Mit Hilfe der in DE 101 46 251 beschriebenen Apparatur wurden diese beiden Lösungen jeweils mit einer Düse gleichzeitig auf eine Glasplatte aufgesprüht, bei 80 °C getrocknet und der Sprühvorgang nach Bildung der jeweiligen Teilschicht mehrmals wiederholt bis eine Schichtdicke der wirkstoffhaltigen Schicht von 200 µm erreicht worden war.

Die auf diese Weise hergestellte wirkstofffreie Schicht weist eine gelbliche Farbe und einen nicht bekömmlichen Geschmack auf.

## Patentansprüche

1. Verfahren zur Herstellung einer filmförmigen Darreichungsform zur oberflächlichen Verabreichung wenigstens eines Wirkstoffes und/oder Nährstoffes an ein Lebewesen umfassend wenigstens eine wirkstoffhaltige und/oder nährstoffhaltige Schicht basierend auf mit mindestens einem Polyacrylsäurederivat vernetzten hydrophilen Polymeren durch Aufbau einzelner Schichten übereinander auf einer glatten Oberfläche,
wobei als Polyacrylsäurederivat eine Polyacrylsäure vernetzt mit Allylsucrose oder Allylpentaerythritol und/oder eine Polyacrylsäure vernetzt mit Divinylglycol, gegebenenfalls mit Calcium neutralisiert, verwendet wird und wobei als hydrophiles Polymer Hydroxypropylmethylcellulose, Hydroxyethylcellulose und/oder Methylcellulose eingesetzt wird.
**gekennzeichnet durch** die Schritte:
a) gleichzeitiges Versprühen einer wäßrigen Lösung der hydrophilen Polymere und des Wirkstoffes und/oder des Nährstoffes und einer wäßrigen Lösung des Polyacrylsäurederivats, wobei die hydrophilen Polymere und das Polyacrylsäurederivat in situ vernetzen,
b) Entfernen des Wassers **durch** Trocknen.

2. Herstellungsverfahren nach Anspruch 1, **dadurch gekennzeichnet, dass** als hydrophiles Polymer Hydroxypropylmethylcellulose eingesetzt wird.

3. Herstellungsverfahren nach einem der Ansprüche 1 oder 2, **dadurch gekennzeichnet, dass** das Gewichtsverhältnis von hydrophilen Polymeren zu Polyacrylsäurederivat (en) 5 : 1 bis 5 : 4 beträgt.

4. Herstellungsverfahren nach einem der Ansprüche 1 oder 2, **dadurch gekennzeichnet, dass** das Gewichtsverhältnis von hydrophilen Polymeren zu Polyacrylsäurederivat (en) 5 : 2 bis 5 : 3 beträgt.

## Claims

1. Process for producing a dosage form in film form for surface administration of at least one active ingredient and/or nutrient to a living creature comprising at least one active ingredient-containing and/or nutrient-containing layer based on hydrophilic polymers crosslinked with at least one polyacrylic acid derivative by building up individual layers successively on a smooth surface, a polyacrylic acid crosslinked with allylsucrose or allylpentaerythritol and/or a polyacrylic acid crosslinked with divinylglycol, where appropriate neutralized with calcium, being used as polyacrylic acid derivative and hydroxypropylmethylcellulose, hydroxyethylcellulose and/or methylcellulose being employed as hydrophilic polymer, **characterized by** the steps:
a) simultaneous spraying of an aqueous solution of the hydrophilic polymers and of the active ingredient and/or of the nutrient and of an aqueous solution of the polyacrylic acid derivative, the hydrophilic polymers and the polyacrylic acid derivative crosslinking in situ,
b) removal of the water by drying.

2. Production process according to Claim 1, **characterized in that** hydroxypropylmethylcellulose is employed as hydrophilic polymer.

3. Production process according to either of Claims 1 and 2, **characterized in that** the weight ratio of hydrophilic polymers to polyacrylic acid derivative(s) is from 5:1 to 5:4.

4. Production process according to either of Claims 1 and 2, **characterized in that** the weight ratio of hydrophilic polymers to polyacrylic acid derivative(s) is from 5:2 to 5:3.

## Revendications

1. Procédé pour la fabrication d'une forme galénique pelliculaire destinée à l'administration superficielle d'au moins une substance active et/ou substance nutritive à un être vivant, comprenant au moins une couche, contenant une substance active et/ou contenant une substance nutritive, à base de polymères hydrophiles réticulés avec au moins un dérivé de poly(acide acrylique), par formation les unes sur les autres de couches individuelles sur une surface lisse, en utilisant comme dérivé de poly(acide acrylique) un poly(acide acrylique) réticulé avec de l'allyl-saccharose ou de l'allylpentaérythritol et/ou un poly(acide acrylique) réticulé avec du divinylglycol, éventuellement neutralisé avec du calcium et en utilisant comme polymère hydrophile de l'hydroxypropyl-méthylcellulose, de l'hydroxyéthylcellulose et/ou de la méthylcellulose,
**caractérisé par** les étapes de :
a) pulvérisation simultanée d'une solution aqueuse des polymères hydrophiles et de la substance active et/ou de la substance nutritive et d'une solution aqueuse du dérivé de poly(acide acrylique), les polymères hydrophiles et le dérivé de poly(acide acrylique) étant réticulés in situ,
b) élimination de l'eau par séchage.

2. Procédé de fabrication selon la revendication 1, **caractérisé en ce qu'**on utilise comme polymère hydrophile l'hydroxypropylméthylcellulose.

3. Procédé de fabrication selon la revendication 1 ou 2, **caractérisé en ce que** le rapport pondéral des polymères hydrophiles au(x) dérivé(s) de poly(acide acrylique) vaut de 5 : 1 à 5 : 4.

4. Procédé de fabrication selon la revendication 1 ou 2, **caractérisé en ce que** le rapport pondéral des polymères hydrophiles au(x) dérivé(s) de poly(acide acrylique) vaut de 5 : 2 à 5 : 3.
